# EUROPÄISCHE PATENTANMELDUNG

(11) **EP 2 990 080 A1**
(43) Veröffentlichungstag der Anmeldung: **02.03.2016**
(21) Anmeldenummer: 15173856.4
(22) Anmeldetag: 25.06.2015
(51) Int. Cl.: A61Q 11/00, A61K 8/97, A61K 8/21

(54) **MUND- UND ZAHNPFLEGE- UND -REINIGUNGSMITTEL ZUR ZAHNFLEISCHVITALISIERUNG**

(30) Priorität: 28.08.2014 DE 102014217216
(71) Anmelder: Henkel AG&Co. KGAA, 40589 Düsseldorf (DE)
(72) Erfinder: GIESEN, Melanie, 47608 Geldern (DE); MIEHLICH, Kristin, 42119 Wuppertal (DE)

(57) **Zusammenfassung**

Mund- und Zahnpflege- und -reinigungsmittel, die - bezogen auf ihr Gewicht - mindestens einen Extrakt aus Quassia *Amara* und 500 bis 1600 ppm Fluorid enthalten, vitalisieren das Zahnfleisch bereits während des Reinigungsvorgangs und stärken seine Widerstandsfähigkeit, so dass auf diese Weise die Entstehung kleiner Mikroverletzungen verhindert und - sollten doch kleine Verletzungen verursacht worden sein - die Regenerationsfähigkeit des Zahnfleisches erhöht wird.

## Beschreibung

Die Erfindung betrifft Zubereitungen zur Mund- und Zahnpflege- und -reinigung, die die Vitalisierung von Zahnfleisch und Mundschleimhaut ermöglichen.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Der Mundraum ist mit einer befeuchteten Schleimhaut (Mucosa) ausgekleidet. Diese besteht generell aus zwei Lagen: einem mehrschichtigen, in der Regel unverhornten Plattenepithel und dem darunter liegenden Bindegewebe. Im Gegensatz zur normalen Haut besitzt die Schleimhaut keine Hornschicht. Als Zahnfleisch wird der epitheliale Bestandteil des Zahnhalteapparates bezeichnet. Auch dieses besteht aus einem mehrschichtigen Plattenepithel, welches ebenfalls nur wenige Hornschichten aufweist.

Ein wichtiges Charakteristikum von Mundschleimhaut und Zahnfleisch ist demzufolge das Fehlen bzw. die sehr schwache Ausbildung der Hornschicht. Mit dieser Hornschicht fehlt der gesamten Mundregion auch eine wichtige äußere Barriere gegenüber mechanischen, chemischen und physikalischen Einflüssen. Die Schleimhautzellen sind somit allen vorgenannten Beanspruchungen ohne Schutzschicht ausgesetzt und reagieren aus diesem Grund empfindlicher auf die durch das Zähneputzen hervorgerufenen mechanischen Beanspruchungen und die mit der Anwendung von Zahncremes verbundenen chemischen Reize.

Der vorliegenden Anmeldung lag daher die Aufgabe zugrunde, ein sowohl für die Zähne als auch für das Zahnfleisch bzw. die Mundschleimhaut optimiertes Zahnreinigungs- und Pflegemittel bereitzustellen, welches den Einsatz der für Zahnreinigung und Kariesprophylaxe unverzichtbaren Inhaltsstoffe wie Abrasiva und Tenside ermöglicht, hierbei aber gleichzeitig deren negativen Einfluss auf das Zahnfleisch minimiert, das Zahnfleisch vitalisiert und dessen Widerstandsfähigkeit stärkt.

Die Zahnreinigung ist auch eine mechanische Beanspruchung, welche - abhängig von der Reinigungstechnik, der Bürstenkonstruktion und des während des Putzvorgangs auf den Bürstenkopf ausgeübten Drucks - dem Zahnfleisch bzw. der Mundschleimhaut kleine Verletzungen zufügen kann. Diese Mikroverletzungen werden vom Verbraucher oftmals gar nicht bemerkt, können aber im schlimmsten Fall zu Schmerzreizen oder Zahnfleischbluten während des Reinigungsvorgangs führen. Je vitaler die Mundschleimhaut bzw. das Zahnfleisch ist, desto widerstandsfähiger ist es gegenüber entsprechenden Mikroverletzungen, und desto schneller und komplikationsloser heilen diese Mikroverletzungen ab.

Es ist daher weiterhin die Aufgabe dieser Erfindung, Zahnreinigungsmittel bereitzustellen, welche das Zahnfleisch bereits während des Reinigungsvorgangs vitalisieren und seine Widerstandsfähigkeit stärken, so dass auf diese Weise die Entstehung kleiner Mikroverletzungen verhindert und - sollten doch kleine Verletzungen verursacht worden sein - die Regenerationsfähigkeit des Zahnfleisches erhöht wird.

In nicht vorhersehbarer Weise wurde nun gefunden, dass die zuvor beschriebenen Aufgaben durch eine Wirkstoffkombination aus Extrakt(en) aus *Quassia Amara* und Fluorid gelöst werden kann.

Gegenstand der vorliegenden Erfindung ist in einer ersten Ausführungsform ein Mund- und Zahnpflege- und -reinigungsmittel, enthaltend - bezogen auf sein Gewicht-
a) mindestens einen Extrakt aus *Quassia Amara,*
b) 500 bis 1600 ppm Fluorid.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes, Mund- und Zahnspülungen sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden. Eine weitere besonders bevorzugte Ausführungsform der vorliegenden Erfindung sind Mundspüllösungen und Mundwasser, die zum Ausspülen der Mundhöhle verwendet werden.

Ein Extrakt im Sinne der vorliegenden Anmeldung ist ein Stoff oder Stoffgemisch, welches durch Extraktion und teilweises oder völliges Eindampfen der Extraktionslösung gewonnen wurde. Man unterscheidet nach der Beschaffenheit *Trockenextrakte* d.h. bis zur Trockene eingedampfte Extrakte, *Fluidextrakte* d.h. mit Lösungsmitteln so hergestellte Extrakte, dass aus einem Teil Droge höchstens 2 Teile Fluid-Extrakt gewonnen werden, *zähflüssige Extrakte bzw. Dickextrakte,* d.h. Extrakte, bei denen ein Teil des Lösungsmittels verdampft wird.

Die in den erfindungsgemäß verwendeten Extrakte können aus den erntefrischen Pflanzen, aber auch aus abgelagerter Ware erhalten werden.

Die Extrakte können mit Wasser, sowie polaren oder unpolaren organischen Lösungsmitteln sowie Mischungen davon in dem Fachmann bekannter Weise hergestellt werden. Die erfindungsgemäß verwendeten Extrakte werden durch Extraktion vorzugsweise mit organischen Lösemitteln, Wasser oder Gemischen daraus, gewonnen. Bevorzugt geeignete organische Lösemittel sind Ketone (z.B. Aceton), Ether, Ester, Alkohole oder halogenierte Kohlenwasserstoffe. Besonders bevorzugte Extraktionsmittel sind Wasser und/oder Alkohole. Unter den Alkoholen sind dabei (C₁ bis C₆)-Alkohole, wie Ethanol und Isopropanol und zwar sowohl als alleiniges Extraktionsmittel als auch in Mischung mit Wasser, bevorzugt. Besonders bevorzugte Extraktionsmittel sind Wasser, Ethanol, 2-Propanol, 1,2-Propylenglykol, 1,3-Butylenglykol, ganz besonders bevorzugt sind Wasser, Ethanol, 2-Propanol und 1,2-Propylenglykol sowie Mischungen hiervon, z.B. eine Mischung 1,2-Propylenglykol/Wasser im Verhältnis 4:1. Extrakte, die durch Extraktion mit Ethanol oder Wasser/Ethanol-Mischungen, erhalten werden können, sind besonders bevorzugt.

Es können sowohl die Extrakte im ursprünglichen Extraktionsmittel als auch Extrakte in Wasser oder anderen organischen Lösungsmitteln und/oder deren Gemisch, insbesondere Ethanol sowie Ethanol/Wasser-Mischungen eingesetzt werden. Bevorzugt wird extrahiertes oder gepresstes Material als Feststoff eingesetzt, dem das Lösungsmittel (insbesondere möglichst schonend) entzogen wurde. Es können aber auch solche Extrakte eingesetzt werden, denen das Lösungsmittel zum Teil entzogen wurde, so dass ein verdickter Extrakt eingesetzt wird. Insbesondere werden die Extrakte in fester Form eingesetzt. Die Extraktion wird bevorzugt bei einer Temperatur von 25°C bis 90°C durchgeführt.

Je nach Wahl der Extraktionsmittel kann es bevorzugt sein, den Extrakt durch Zugabe eines Lösungsvermittlers zu stabilisieren. Als Lösungsvermittler geeignet sind z. B. Ethoxylierungsprodukte von gegebenenfalls gehärteten pflanzlichen und tierischen Ölen. Bevorzugte Lösungsvermittler sind ethoxylierte Mono-, Di- und Triglyceride von C8-22-Fettsäuren mit 4 bis 50 Ethylenoxid-Einheiten, z. B. hydriertes ethoxyliertes Castoröl, Olivenölethoxylat, Mandelölethoxylat, Nerzölethoxylat, Polyoxyethylenglykolcapryl-/caprinsäureglyceride, Polyoxyethylenglycerinmonolaurat und Polyoxyethylenglykolkokos-fettsäureglyceride. Besonders bevorzugt ist Olivenölethoxylat (INCI-Bezeichnung: PEG-10 Olive Glycerides).

Die Trockenmasse des Extraktes ist abhängig von der Molmasse und der Löslichkeit der dispergierten Inhaltsstoffe und beträgt in der Regel, jeweils bezogen auf das Gewicht des Extraktes, 1 bis 80 Gew.-%. Bevorzugt beträgt die Trockenmasse 15 bis 50 Gew.-% und besonders bevorzugt 20 bis 35 Gew.-%. Bei einem Molekulargewicht der Inhaltsstoffe über 100.000 Dalton kann eine Trockenmasse von 1 bis 20 Gew.-% bevorzugt und eine Trockenmasse von 1 bis 10 Gew.-% besonders bevorzugt sein. Besonders bevorzugte kosmetische Mittel sind dadurch gekennzeichnet, dass die Trockenmasse des Extraktes 5 - 80 Gew.-%, bezogen auf das Gewicht des Extraktes, beträgt.

Als ersten wesentlichen Inhaltsstoff enthalten die erfindungsgemäßen Zusammensetzungen mindestens einen Extrakt aus *Quassia Amara.*

Der *Quassia Amara,* Brasilianischer Quassiabaum, Quassiabaum, Quassiaholzbaum, Bitterquassia oder wie einige andere Arten auch Bitterholz genannt, ist eine Pflanzenart in der Familie der Bittereschengewächse (Simaroubaceae). Sie ist in der Neotropis weit verbreitet. Die erfindungsgemäß eingesetzten Extrakte werden vorzugsweise aus dem Holz, Blättern, Blüten, Früchten, besonders bevorzugt aus dem Holz gewonnen.

Extrakt aus Quassia Amara können nach bekannten Herstellungsverfahren in variabler Zusammensetzung mit Lösungsmitteln wie z. B. Wasser, Methanol, Ethanol, Aceton, etc., und deren Gemische bei Temperaturen von Raumtemperatur bis 100 °C unter gelinder bis heftiger Durchmischung innerhalb von 10 Min. bis 24 Std. unter Normaldruck bis zu 200 bar erhalten werden. Zur Anreicherung wirksamkeitsbestimmender Komponenten können weitere Konzentrierungsschritte durchgeführt werden wie z. B. flüssig-flüssig-Verteilung mit z. B. 1-Butanol/Wasser oder Ethylacetat/Wasser, Adsorption-Desorption an Ionenaustauscher, LH20, HP20 und andere Harze oder chromatographische Abtrennungen über RP18, Kieselgel, etc.. Falls die Weiterverarbeitung zu Trockenextrakten erwünscht ist, erfolgt diese nach an sich bekannten Verfahren durch Abziehen des Lösungsmittels bei erhöhter Temperatur und / oder reduziertem Druck. Die Angaben zum Gewichtsanteil von *Quassia Amara* am Gesamtgewicht der erfindungsgemäßen Mittel beziehen sich auf die Trockenmasse des Extraktes.

Erfindungsgemäß ganz bevorzugt ist ein Extrakt, der durch Extraktion von *Quassia Amara* Holz mit Wasser erhältlich ist. Dieser ist auch unter der INCI-Bezeichnung QUASSIA AMARA WOOD EXTRACT bekannt.

Das Handelsprodukt des Extraktes aus *Quassia Amara* kann außer Extraktionsmittel noch zusätzliche Lösungsmittel zur Verbesserung der Haltbarkeit des Produkts enthalten. Propylenglycol, 1,3 - Butylenglycol und Pentylenglycol sind beispielweise solche antimikrobiellen Lösungsmittel.

Erfindungsgemäß bevorzugte Mittel setzen den/die Extrakt(e) aus *Quassia Amara* innerhalb enger Mengenbereiche ein. Hier sind erfindungsgemäße Mund- und Zahnpflege- und -reinigungsmittel bevorzugt, die - bezogen auf ihr Gewicht - 0,001 - 5 Gew.-%, vorzugsweise 0,001 - 4 Gew.-%, besonders bevorzugt 0,002 - 2 Gew.-%, außerordentlich bevorzugt 0,003 - 1 Gew.-% und insbesondere 0,005 bis 0,5 Gew.-% Extrakt(e) aus *Quassia Amara* enthalten.

Es kann in einer anderen Ausführungsform auch bevorzugt sein, dass der Extrakt mindestens ein polares Lösungsmittel, ausgewählt aus Ethanol, 1-Propanol, 2-Propanol, 1-Butanol, 2-Butanol, Ethylenglykol, 1,2-Propylenglykol, 1,3-Butylenglykol, Glycerin und Wasser, sowie Mischungen hiervon enthält.

Überraschenderweise wurde gefunden, dass *Quassia Amara* die Collagensynthese in Gingiva-Fibroblasten und die ATP-Synthese von Gingiva-Keratinozyten anregt, was die Vitalität der Zellen fördert. Dies spielt eine besondere Rolle für die Mundhygiene, da bei Entzündungen des Zahnfleisches im Rahmen einer Gingivitis-Erkrankung die unterliegende Matrix, die zum großen Teil aus Collagen besteht, abgebaut wird, und sich damit das Zahnfleisch zurückzieht. Darüber hinaus ist es von Vorteil, wenn die Collagenproduktion im Zahnfleisch angeregt wird, da das Zahnfleisch dadurch kräftiger wird und besser gegen Entzündungen geschützt wird. Darüber hinaus ist bekannt, dass das Zahnfleisch u.a. aufgrund zurückgehender Matrixbestandteile in den tieferen Schichten im Alter zurückgeht und dadurch auch anfälliger für Entzündungen wird. Durch das "Auffüllen" von natürlichen Matrixbestandteilen (Collagen), kann diesem Prozess vorgebeugt werden. Darüber hinaus ist die Anregung der ATP-Synthese vorteilhaft, da insbesondere das Zahnfleisch anfällig für Entzündungen ist und die Widerstandsfähigkeit des Zahnfleisches durch die Förderung des Zellmetabolismus erhöht werden kann.

Als zweiten wesentlichen Inhaltsstoff enthalten die erfindungsgemäßen Mittel Fluorid. Dieses kann in Form anorganischer Fluoridsalze (Natriumfluorid, Zinn(II)fluorid, Natriummonofluorphosphat usw.) bereitgestellt werden, auch Aminfluoride wie Olaflur sind geeignet.

Überraschenderweise wurde gefunden, dass die Fluoriddeposition gesteigert werden kann, wenn oberhalb bestimmter Fluoridgehalte zusätzlich Extrakt(e) aus *Quassia Amara* in den Zusammensetzungen enthalten sind. Die Mindestmenge an Fluorid beträgt dabei 500 ppm, unterhalb dieser Grenze macht sich der Einsatz von Extrakt(en) aus *Quassia Amara* auf die Fluoriddeposotion nicht bemerkbar. Besonders gute Fluoriddepositionswerte werden erzielt, wenn der/die Extrakt(e) aus *Quassia Amara* in Gegenwart höherer Fluoriddmengen eingesetzt werden, wobei sich Werte von 1000 ppm Fluorid und darüber als besonders bevorzugt gezeigt haben.

Besonders bevorzugte erfindungsgemäße Mund- und Zahnpflege- und reinigungsmittel sind dadurch gekennzeichnet, dass sie 1225 bis 1575 ppm, vorzugsweise 1250 bis 1550 ppm, weiter bevorzugt 1275 bis 1525 ppm, noch weiter bevorzugt 1300 bis 1500 ppm, noch weiter bevorzugt 1325 bis 1475 ppm und insbesondere 1350 bis 1450 ppm Fluorid enthalten.

Wird Fluorid in Form von Natriumfluorid bereitgestellt, entspricht 1 Gew.-% Natriumfluorid ungefähr 4524 ppm Fluorid, so dass bevorzugte erfindungsgemäße Mittel 0,27 bis 0,35 Gew.-%, vorzugsweise 0,28 bis 0,34 Gew.-%, weiter bevorzugt 0,29 bis 0,33 Gew.-% und insbesondere 0,30 bis 0,32 Gew.-% Natriumfluorid enthalten.

Die erfindungsgemäßen Mund- und Zahnpflege- und -reinigungsmittel enthalten weitere Inhaltsstoffe. Bevorzugt ist hierbei der Einsatz von sogenannten Feuchthaltemitteln, die bei Zahnpasten das Austrocknen verhindern und die Wirksamkeit der erfindungsgemäßen Kombination steigern. Bei sogenannten Flüssigzahncremes mit fließfähiger Rheologie dienen diese als Matrix und werden in höheren Mengen eingesetzt. Bei Mundwässern und Mundspülungen dienen diese Alkohole als Konsistenzregler und zusätzliche Süßungsmittel.

Hier sind erfindungsgemäße Mund- und Zahnpflege- und -reinigungsmittel bevorzugt, die - bezogen auf ihr Gewicht - 0,5 bis 60 Gew.-%, vorzugsweise 0,75 bis 55 Gew.-%, besonders bevorzugt 1 bis 50 Gew.-% und insbesondere 2 bis 40 Gew.-% mindestens eines mehrwertigen Alkohols aus der Gruppe Sorbit und/oder Glycerin und/oder 1,2-Propylenglycol.-% oder deren Mischungen enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1:(0,5-1):(0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Es hat sich gezeigt, dass Glycerin und Sorbitol in bestimmten Mengen die Vitalisierung des Zahnfleisches durch die erfindungsgemäße Kombination noch weiter positiv beeinflussen können. Erfindungsgemäße Mund und Zahnpflege- und -reinigungsmittel, die
i) 0 bis 15 Gew.-% Sorbitol
ii) 0 bis 30 Gew.-% Glycerin,
enthalten, mit der Maßgabe, dass die Gesamtmenge an Inhaltsstoff(en) aus den Gruppen i) und ii) 5 bis 40 Gew.-% beträgt, sind besonders bevorzugte Ausführungsformen der vorliegenden Erfindung.

Besonders bevorzugt ist der Einsatz von Sorbit, so dass Mittel, die außer Sorbit keine anderen mehrwertigen Alkohole enthalten, besonders bevorzugt sind.

Als weiteren Inhaltsstoff können die erfindungsgemäßen Mittel Tensid(e) enthalten. Diese steigern die Verfügbarkeit der erfindungsgemäßen Wirkstoffkombination am Zahn und Zahnfleisch. Auch die Tenside werden vorzugsweise innerhalb enger Mengenbereiche eingesetzt, so dass bevorzugte erfindungsgemäße Mund und Zahnpflege- und -reinigungsmittel dadurch gekennzeichnet sind, dass sie 0,1 bis 10 Gew.-%, vorzugsweise 0,2 bis 8 Gew.-%, weiter bevorzugt 0,5 bis 7 Gew.-%, noch weiter bevorzugt 1 bis 5 Gew.-% und insbesondere 1,5 bis 3 Gew.-% Tensid(e) enthalten.

Geeignete Tenside sind z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂-C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆ )-estern, Acylsarcosinen, Acyltauriden und Acylisothionaten mit jeweils 8-18 C-Atomen in der Acylgruppe. Auch zwitterionische, ampholytische und nichtionische Tenside sind geeignet.

Als nichtionische Tenside können prinzipiell alle wasserlöslichen Tenside eingesetzt werden, die eine bevorzugt lineare Alkyl- oder Acylgruppe mit 12-18 C-Atomen und eine die Wasserlöslichkeit bewirkende Polyol- oder Polyethergruppe tragen. Solche Tenside sind z. B. die Anlagerungsprodukte von Ethylenoxid an Fettalkohole, an Fettsäuren, an Fettsäurealkanolamide, an Fettsäuremonoglyceride, an Fettsäurediglyceride, an Rhizinusöl, an Sorbitanmonofettsäureester oder an Methylglycosid-monofettsäureester. Andere geeignete nichtionogene Tenside sind die linearen Alkyl(C₈-C₁₆-)-Glucoside und Alkyl(C₈-C₁₆-)-Oligoglucoside und deren Ethylenoxidanlagerungsprodukte. Die Wasserlöslichkeit der Ethylenoxidaddukte kann durch die Menge des angelagerten Ethylenoxids erhöht werden, sie sollte wenigstens so hoch sein, daß sich 1 Gew.-% des Tensids in Wasser bei 20°C löst.

Erfindungsgemäß bevorzugte Mund und Zahnpflege- und -reinigungsmittel sind dadurch gekennzeichnet, dass sie 0,1 bis 5 Gew.-%, vorzugsweise 0,2 bis 4,0 Gew.-%, besonders bevorzugt 0,5 bis 3,5 Gew.-%, weiter bevorzugt 0,75 bis 3,0 Gew.-% und insbesondere 1 bis 2,5 Gew.-% nichtionische(s) Tensid(e) enthalten.

Eine weitere bevorzugt einzusetzende Gruppe von Tensiden stellen die anionischen Tenside dar. Erfindungsgemäß bevorzugte Mund und Zahnpflege- und -reinigungsmittel sind dadurch gekennzeichnet, dass sie 0 bis 5 Gew.-%, vorzugsweise 0,1 bis 4,5 Gew.-%, besonders bevorzugt 0,5 bis 4 Gew.-%, weiter bevorzugt 0,75 bis 3,5 Gew.-% und insbesondere 1 bis 3 Gew.-% anionische(s) Tensid(e) enthalten.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkyl-sulfosuccinate, Mono-und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Ganz besonders bevorzugte erfindungsgemäße Mittel enthalten Alkylsulfat(e) als anionisches Tensid. Hier sind erfindungsgemäß besonders bevorzugte Mund und Zahnpflege- und - reinigungsmittel sind dadurch gekennzeichnet, dass sie 0,1 bis 4,0 Gew.-%, vorzugsweise 0,75 bis 3,0 Gew.-%, besonders bevorzugt 1,0 bis 2,5 Gew.-%, weiter bevorzugt 1,25 bis 2,0 Gew.-% und insbesondere 1,5 bis 1,8 Gew.-% Natriumlaurylsulfat enthalten.

Mit besonderem Vorzug enthalten die erfindungsgemäßen Mittel zusätzlich oder alternativ zu den anionischen Tensiden amphotere(s) Tensid(e). Unter ampholytischen Tensiden und Emulgatoren werden solche oberflächenaktiven Verbindungen verstanden, die außer einer C₈- C₂₄- Alkyl- oder - Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Besonders bevorzugte erfindungsgemäße Mund und Zahnpflege- und -reinigungsmittel sind dadurch gekennzeichnet, dass sie es 0,01 bis 2 Gew.-%, vorzugsweise 0,05 bis 1,5 Gew.-%, weiter bevorzugt 0,1 bis 1 Gew.-%, noch weiter bevorzugt 0,12 bis 0,7 Gew.-% und insbesondere 0,15 bis 0,6 Gew.-% amphotere(s) Tensid(e) enthalten.

Besonders bevorzugte erfindungsgemäße Mund und Zahnpflege- und -reinigungsmittel sind dadurch gekennzeichnet, dass sie amphotere(s) Tensid(e) aus den Gruppen der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionat,
- Kokosacylaminoethylaminopropionat
- C₁₂ - C₁₈ - Acylsarcosin,
- N-Alkyl-N,N-dimethylammonium-glycinate, beispielsweise Kokosalkyl-dimethyl-ammoniumglycinat,
- N-Acyl-aminopropyl-N,N-dimethylammoniumglycinate, beispielsweise Kokosacylaminopropyl-dimethylammoniumglycinat,
- 2-Alkyl-3-carboxymethyl-3-hydroxyethyl-imidazoline mit jeweils 8 bis 18 C-Atomen in der Alkyl- oder Acylgruppe
- Kokosacylaminoethylhydroxyethylcarboxymethylglycinat
- der unter der INCI-Bezeichnung Cocamidopropyl Betain bekannten Verbindungen,
- der unter der INCI-Bezeichnung Disodium Cocoamphodiacetate bekannten Verbindungen
enthalten.

Besonders bevorzugte Mund und Zahnpflege- und -reinigungsmittel enthalten als amphotere Tenside Betaine der Formel (Bet-I) 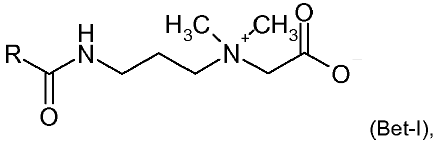 in der R für einen geradkettigen oder verzweigten, gesättigten oder ein- bzw. mehrfach ungesättigten Alkyl- oder Alkenlyrest mit 8 bis 24 Kohlenstoffatomen steht.

Diese Tenside werden nach der INCI-Nomenklatur als Amidopropylbetaine bezeichnet, wobei die Vertreter, die sich von Kokosfettsäuren ableiten, bevorzugt sind und als Cocoamidopropylbetaine bezeichnet werden. Besonders bevorzugt werden erfindungsgemäß Tenside der Formel (Bet-I) eingesetzt, die ein Gemisch der folgenden Vertreter sind:

H₃C-(CH₂)₇-C(O)-NH-(CH₂)₃N⁺(CH₃)₂CH₂COO⁻

H₃C-(CH₂)₉-C(O)-NH-(CH₂)₃N⁺(CH₃)₂CH₂COO⁻

H₃C-(CH₂)₁₁-C(O)-NH-(CH₂)₃N⁺(CH₃)₂CH₂COO⁻

H₃C-(CH₂)₁₃-C(O)-NH-(CH₂)₃N⁺(CH₃)₂CH₂COO⁻

H₃C-(CH₂)₁₅-C(O)-NH-(CH₂)₃N⁺(CH₃)₂CH₂COO⁻

H₃C-(CH₂)₇-CH=CH-(CH₂)₇-C(O)-NH-(CH₂)₃N⁺(CH₃)₂CH₂COO⁻

Besonders bevorzugt werden Tenside der Formel (Bet-I) innerhalb engerer Mengenbereiche eingesetzt. Hier sind erfindungsgemäße Mund und Zahnpflege- und -reinigungsmittel bevorzugt, die - bezogen auf ihr Gewicht - 0,1 bis 4,0 Gew.-%, vorzugsweise 0,2 bis 3,0 Gew.-%, bsonders bevorzugt 0,3 bis 2,5 Gew.-%, weiter bevorzugt 0,4 bis 2,0 Gew.-% und insbesondere 0,5 bis 1,0 Gew.-% Cocamidopropylbetain enthalten.

Erfindungsgemäße Mittel, die sowohl anionische(s) als auch amphotere(s) Tensid(e) enthalten, sind wegen der verbesserten Wirksamkeit der erfindungsgemäßen Kombination noch weiter bevorzugt. Ganz besonders bevorzugte erfindungsgemäße Mund und Zahnpflege- und - reinigungsmittel sind dabei dadurch gekennzeichnet, dass sie sowohl anionische(s) als auch amphotere(s) Tensid(e) enthalten und das Gewichtsverhältnis von anionischen Tensiden zu amphoteren Tensiden bei ≥ 1:1, vorzugsweise bei ≥ 2,5:1, besonders bevorzugt bei ≥ 4:1 , weiter bevorzugt bei ≥ 5:1 , und insbesondere bei ≥ 7:1 liegt.

Zur Entfaltung der Reinigungsleistung können erfindungsgemäße Mittel Poliermittel enthalten. Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 - 50 Gew.-% des Zahnpflegemittels.

Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wässrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die sogenannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15-110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1:1)-Dispersion von 30-60 Pa.s (20°C) in einer Menge von 10-20 Gew.-% der Zahnpaste. Bevorzugt geeignete Fällungskieselsäuren dieser Art weisen außerdem gerundete Ecken und Kanten auf und sind unter der Handelsbezeichnung Sident^{®}12 DS (DEGUSSA) erhältlich.

Andere Fällungskieselsäuren dieser Art sind Sident^{®} 8 (DEGUSSA) und Sorbosil^{®} AC 39 (Crosfield Chemicals). Diese Kieselsäuren zeichnen sich durch eine geringere Verdickungswirkung und eine etwas höhere mittlere Teilchengröße von 8-14 µm bei einer spezifischen Oberfläche von 40-75 m²/g (nach BET) aus und eignen sich besonders gut für flüssige Zahncremes. Diese sollten eine Viskosität (25°C, Scherrate D = 10 s⁻¹) von 10-100 Pa.s aufweisen.

Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1-3 µm. Solchen Zahnpasten setzt man daher bevorzugt neben den genannten Fällungskieselsäuren noch feinteiligere, sogenannte Verdickungskieselsäuren mit einer BET-Oberfläche von 150-250 m²/g zu, z.B. die Handelsprodukte Sipernat 22 LS oder Sipernat^{®} 320 DS. Als weitere Poliermittelkomponente kann auch z.B. Aluminiumoxid in Form von schwach calcinierter Tonerde mit einem Gehalt an Aluminiumoxid in einer Menge von ca. 1-5 Gew.-% enthalten sein. Ein solches geeignetes Aluminiumoxid ist beispielsweise unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Die angegebenen Mengen beziehen sich auf die Gesamtmenge an Reibkörpern, wobei einzelne Reibkörper vorzugsweise in engeren Mengenbereichen eingesetzt werden. Erfindungsgemäß bevorzugte Mittel enthalten beispielsweise 5 bis 20 Gew.-%, vorzugsweise 8 bis 21 Gew.-%, weiter bevorzugt 9 bis 20 Gew.-% und insbesondere 11 bis 19 Gew.-% Kieselsäure(n). Weiter bevorzugte erfindungsgemäße Mittel sind dadurch gekennzeichnet, dass sie 0,25 bis 2 Gew.-%, vorzugsweise 0,5 bis 1,5 Gew.-% und insbesondere 0,75 bis 1,25 Gew.-% Aluminiumoxid enthalten.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper,vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Es hat sich im Hinblick auf die Fluoriddeposition als vorteilhaft herausgestellt, die Mengen an Tensid(en) und Poliermittel(n) aufeinander abzustimmen. Erfindungsgemäß bevorzugt beträgt das Gewichtsverhältnis von Poliermittel(n) zu Tensid(en) ≤ 6, d.h. die Poliermittel werden maximal im 6-fachen (Gewichts-)Überschuss zu den Tensiden eingesetzt. Das Gewichtsverhältnis bezieht sich dabei auf das Gewichtsverhältnis aller Stoffe aus den genannten Gruppen zueinander, wobei nur die Aktivsubstanzen gerechnet werden. Enthält ein erfindungsgemäßes Mittel z.B. 12 Gew.-% Poliermittel und 4 Gew.-% einer 50%igen Tensidlösung, so beträgt das Gewichtsverhältnis 12 / 2 = 6. Enthält ein erfindungsgemäßes Mittel z.B. 12 Gew.-% Poliermittel und 4 Gew.-% einer 50%igen Tensidlösung sowie 1 Gew.-% reines Natriumdodecylsulfat, so beträgt das Gewichtsverhältnis 12 / (2+1)=4.

Besonders gute Werte wurden für Mund und Zahnpflege- und -reinigungsmittel erreicht, bei denen das Gewichtsverhältnis von Poliermittel(n) zu Tensid(en) im Bereich ≥ 5 bis ≤ 15, vorzugsweise im Bereich ≥ 7,5 bis ≤ 12,5, weiter bevorzugt im Bereich ≥ 10 bis ≤ 12 und insbesondere im Bereich ≥ 10,25 bis ≤ 11,9 liegt.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z.B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Als besonders veträglich mit der erfindungsgemäßen Wirkstoffkombination haben sich CMC und Xanthan erwiesen. Bei Einssatz dieser Verdickern ist die erfindungsgemäße Wirkung besonders ausgeprägt. Erfindungsgemäß besonders bevorzugte Mund- und Zahnpflege- und - reinigungsmittel sind demnach dadurch gekennzeichnet, dass sie - bezogen auf ihr Gewicht - 0,2 bis 7,5 Gew.-%, vorzugsweise 0,25 bis 5 Gew.-%, besonders bevorzugt 0,3 bis 4 Gew.-%, weiter bevorzugt 0,4 bis 3 Gew.-%, noch weiter bevorzugt 0,45 bis 2 Gew.-% und insbesondere 0,5 bis 0,8 Gew.-% Carboxymethylcellulose enthalten.

Weitere besonders bevorzugte Mund- und Zahnpflege- und -reinigungsmittel sind dadurch gekennzeichnet, dass sie - bezogen auf ihr Gewicht - 0,15 bis 5 Gew.-%, vorzugsweise 0,2 bis 2,5 Gew.-%, besonders bevorzugt 0,25 bis 1 Gew.-%, weiter bevorzugt 0,3 bis 0,75 Gew.-%, noch weiter bevorzugt 0,35 bis 0,6 Gew.-% und insbesondere 0,4 bis 0,5 Gew.-% Xanthan enthalten. Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Maltose, Fructose.

Im Rahmen der vorliegenden Erfindung wurde überraschend festgestellt, dass die erste maßgebliche Komponente der erfindungsgemäßen Mund- und Zahnpflege- und - reinigungsmittels, ein Extrakt aus *Quassia Amara* eine herausragende Eignung zur Vitalisierung der Zellen von Zahnfleisch und Mundschleimhaut besitzt.

Die erfindungsgemäßen Mittel können in einem kosmetischen, nicht therapeutischen Verfahren zur Reinigung der Zähne bei gleichzeitiger Vitalisierung bzw. Kräftigung des Zahnfleisches und der Mundschleimhaut eingesetzt werden

Ein weiterer Gegenstand der vorliegenden Erfindung ist ein Verfahren zum Reinigen von Zähnen, bei dem ein erfindungsgemäßes Mittel auf den Bürstenkopf einer Zahnbürste aufgetragen und mit der Zahnbürste die Zähne geputzt werden.

Die Zahnfleischvitalisierung gelingt insbesondere mit elektrischen Zahnbürsten nochmals besser. Ein weiterer Gegenstand der vorliegenden Erfindung ist daher ein Verfahren zur Zahnreinigung, gekennzeichnet durch die Schritte
a) Bereitstellung einer Zahnbürste, deren Bürstenkopf elektrisch in Bewegung versetzt werden kann;
b) Applikation von 0,5 bis 5 g eines erfindungsgemäßen Mittels auf den Bürstenkopf,
c) 30- bis 300-sekündiges Zähneputzen mit dem erfindungsgemäßen Mittel unter Einsatz des elektrisch in Bewegung versetzten Bürstenkopfes.

Durch Applikation von Extrakt(en) aus *Quassia Amara* auf den Zellen von Zahnfleisch bzw. Mundschleimhaut erhöht sich deren metabolische Stoffwechselaktivität. Den Zellen steht demzufolge mehr Energie zur Verfügung, und die Collagenproduktion in den Zellen kann gesteigert werden, was eine Kräftigung des Zahnfleisches zur Folge hat.

Ein weiterer Gegenstand der vorliegenden Erfindung ist daher die nicht-therapeutische, kosmetische Verwendung von Extrakten aus *Quassia Amara*
- zur Steigerung der Collagensynthese in Gingiva- Fibroblasten und/oder
- zur Steigerung der ATP-Synthese von Gingiva-Keratinozyten und/oder
- zur Vitalisierung und Kräftigung des Zahnfleischs und der Mundschleimhaut und/oder
- zur Verbesserung der Zahnfleischregeneration und/oder
- zur Verjüngung des Zahnfleisches und/oder
- zum Schutz vor Zahnfleischentzündungen und/oder
- zur Glättung der Zahnschmelzoberflächen und/oder
- zur Reparatur mikroskopisch feiner Fissuren im Zahnschmelz und/oder
- zur Senkung der Zahnempfindlichkeit und/oder
- zur Senkung der Zahnempfindlichkeit gegen süße Speisen und Getränke und/oder
- zur Senkung der Zahnempfindlichkeit gegen saure Speisen und Getränke und/oder
- zur Senkung der Zahnempfindlichkeit gegen heiße Speisen und Getränke und/oder
- zur Senkung der Zahnempfindlichkeit gegen kalte Speisen und Getränke und/oder
- zur Senkung der Zahnempfindlichkeit gegen taktile Reize.

Die genannten Effekte lassen sich mit den erfindungsgemäßen Mitteln besonders gut erreichen. Ein weiterer Gegenstand der vorliegenden Erfindung ist daher die nicht-therapeutische, kosmetische Verwendung eines erfindungsgemäßen Mund und Zahnpflege- und -reinigungsmittels
- zur Steigerung der Collagensynthese in Gingiva- Fibroblasten und/oder
- zur Steigerung der ATP-Synthese von Gingiva-Keratinozyten und/oder
- zur Vitalisierung und Kräftigung des Zahnfleischs und der Mundschleimhaut und/oder
- zur Verbesserung der Zahnfleischregeneration und/oder
- zur Verjüngung des Zahnfleisches und/oder
- zum Schutz vor Zahnfleischentzündungen und/oder
- zur Glättung der Zahnschmelzoberflächen und/oder
- zur Reparatur mikroskopisch feiner Fissuren im Zahnschmelz und/oder
- zur Senkung der Zahnempfindlichkeit und/oder
- zur Senkung der Zahnempfindlichkeit gegen süße Speisen und Getränke und/oder
- zur Senkung der Zahnempfindlichkeit gegen saure Speisen und Getränke und/oder
- zur Senkung der Zahnempfindlichkeit gegen heiße Speisen und Getränke und/oder
- zur Senkung der Zahnempfindlichkeit gegen kalte Speisen und Getränke und/oder
- zur Senkung der Zahnempfindlichkeit gegen taktile Reize.

Bezüglich bevorzugter Ausführungsformen des erfindungsgemäßen Verfahrens und der erfindungsgemäßen Verwendung gilt mutatis mutandis das zu den erfindungsgemäßen Mitteln Gesagte.

### Beispiele:

### Beispiel 1

### Steigerung der Zellteilung

Als Maß für die Vitalität wird die Zellteilungsaktivität von Gingiva Fibroblasten nach Inkubation mit einem Extrakt aus Quassia Amara Wood gemessen. Ein fluoreszierendes Farbstoffmolekül wird der Zellkultur beigesetzt und mit den Zellen inkubiert. Dabei dringt der Farbstoff in die Zellen ein und lagert sich an die DNA an. Ein potenziell vitalisierender Bioaktivstoff kann die Zellteilung anregen. Je höher die Zellzahl in der Kultur ist, desto mehr DNA wird angefärbt und kann spektroskopisch vermessen.

Die Ergebnisse zeigen, dass die Zugabe von 0,01 Gew-% Quassia Amara Wood Extrakt in Gingiva Zellen zu einer signifikanten Steigerung der DNA-Menge und damit Vitalität führte.

**Tabelle 1: Einfluss von Quassia Amara Wood Extrakt auf die Zellzahl**

| | Mittelwert (MW) | Standard Deviation (SD) |
|---|---|---|
| Zellkultur ohne Quassia Amara Wood Extrakt | 100,0 | 27,4 |
| Zellkultur mit 0,01 Gew.-% Quassia Amara Wood Extrakt | 160,7 | 40,79 |

### Beispiel 2

### Steigerung der Collagensynthese

Die Expression der matrixspezifischen Strukturmoleküle Collagen I und Collagen III kann an kultivierten Gingiva-Fibroblasten mit Hilfe eines quantitativen Real-Time-PCR-Verfahrens untersucht werden. Zur Durchführung der PCR wird zunächst mit Hilfe des RNeasy Mini Kits der Fa. Qiagen die RNA aus den Zellen isoliert und mittels reverser Transkription in cDNA umgeschrieben. Bei der anschließend PCR Reaktion, die mit Hilfe genspezifischer Primer für Collagen III durchgeführt wird und die der Amplikation der gesuchten Genabschnitte dient, wird die Bildung der PCR-Produkte online über ein Fluoreszenzsignal detektiert. Das Fluoreszenzsignal ist dabei proportional zur Menge des gebildeten PCR-Produktes. Je stärker die Expression eines bestimmten Gens ist, desto größer ist die Menge an gebildetem PCR-Produkt und um so höher ist das Fluoreszenzsignal.

Zur Quantifizierung der Genexpression wird die unbehandelte Kontrolle gleich 100% gesetzt und die Expression der zu bestimmenden Gene darauf bezogen.

Quassia Amara Wood Extrakt steigerte die Genexpression von Collagen I um 120% und von Collagen III um 40%.

**Tabelle 2: Einfluss von Quassia Amara Wood Extrakt auf die Collagensynthese**

| | Collagen I [%] | Collagen III [%] |
|---|---|---|
| Unbehandelt | 100 | 100 |
| 0,01 Gew.-% Quassia Amara Wood Extrakt | 220 | 140 |

### Beispiel 3: Formulierungen:

Es wurden die folgenden Formulierungen hergestellt, wobei alle Zahlenwerte in den nachfolgenden Beispielen - sofern nicht anders angegeben - der Menge des jeweiligen Wirkstoffs in Gewichtsprozent bezogen auf das Gesamtgewicht des Mittels entsprechen.

### Zahncremeformulierungen:

| **Rohstoff** | **Bsp. 1** | **Bsp. 2** | **Bsp. 3** |
|---|---|---|---|
| Sorbitol | 45 | 60 | 50 |
| Hydrated Silica | 10 | 10 | 10 |
| Saccharin (Natriumsalz) | 0,5 | 0,5 | 0,5 |
| Quassia Amara Wood Extrakt | 0,001 | 0,01 | 0,1 |
| Natriumfluorid | 0,32 | 0,32 | 0,32 |
| Sodium Lauryl Sulfat | 1,0 | 1,0 | 0,9 |
| Cocoamidopropylbetain | --- | --- | 0,5 |
| Aroma | 1,0 | 1,0 | 1,0 |
| Ethanol | --- | 1,5 | --- |
| Xanthan Gum | 0,5 | 0,5 | 0,5 |
| Maleic anhydride, methyl vinyl ether copolymer | 1,0 | 1,0 | 1,0 |
| Wasser | ad 100 | ad 100 | ad 100 |
| | | | |

| **Rohstoff** | **Bsp. 4** | **Bsp. 5** | **Bsp. 6** |
|---|---|---|---|
| Sorbitol | 50 | 50 | 50 |
| Hydrated Silica | 10 | 10 | 10 |
| Saccharin (Natriumsalz) | 0,5 | 0,5 | 0,5 |
| Quassia Amara Wood Extrakt | 0,01 | 0,1 | 0,5 |
| Natriumfluorid | 0,32 | 0,32 | 0,32 |
| Sodium Laureth Sulfate (Sodium lauryl ether sulfate (2 EO)) | 1,0 | 1,0 | 0,9 |
| Cocoamidopropylbetain | --- | --- | 0,6 |
| Aroma | 1,0 | 1,0 | 1,0 |
| Ethanol | --- | 1,5 | --- |
| Xanthan Gum | 0,5 | 0,5 | 0,5 |
| Maleic anhydride, methyl vinyl ether copolymer | 1,0 | 1,0 | 1,0 |
| Wasser | ad 100 | ad 100 | ad 100 |
| | | | |

| **Rohstoff** | **Bsp. 7** | **Bsp. 8** | **Bsp. 9** |
|---|---|---|---|
| Sorbitol | 60 | 60 | 60 |
| Hydrated Silica | 20 | 20 | 20 |
| Saccharin (Natriumsalz) | 0,5 | 0,5 | 0,5 |
| Quassia Amara Wood Extrakt | 0,01 | 0,1 | 0,5 |
| Natriumfluorid | 0,32 | 0,32 | 0,32 |
| Sodium Laureth Sulfate (Sodium lauryl ether sulfate (2 EO)) | 1,0 | 1,0 | 1,0 |
| Cocoamidopropylbetain | 0,2 | 0,3 | 0,5 |
| Aroma | 1,0 | 1,0 | 1,0 |
| Ethanol | --- | 1,5 | --- |
| Xanthan Gum | 0,5 | 0,5 | 0,5 |
| Maleic anhydride, methyl vinyl ether copolymer | 1,0 | 1,0 | 1,0 |
| Wasser | ad 100 | ad 100 | ad 100 |

### Mundwasser-Formulierunpen:

| **Rohstoff** | **Bsp. 10** | **Bsp. 11** | **Bsp. 12** |
|---|---|---|---|
| Sorbitol | 2,5 | 3,0 | 2,5 |
| PEG-60 Hydrogenated Castor Oil | 0,5 | 0,5 | 0,5 |
| Trinatriumcitrat Dihydrat | 0,4 | 0,4 | 0,4 |
| Citronensäure | 0,001 | 0,05 | 0,1 |
| Saccharin (Natriumsalz) | 0,03 | 0,1 | 0,3 |
| Cetylpyridiniumchlorid | 0,01 | 0,05 | 0,1 |
| Quassia Amara Wood Extrakt | 0,001 | 0,01 | 0,1 |
| Natriumfluorid | 0,32 | 0,32 | 0,32 |
| Sodium Lauryl Sulfat | --- | 0,2 | --- |
| Sodium Laureth Sulfate | 0,2 | --- | 0,1 |
| (Sodium lauryl ether sulfate (2 EO)) | | | |
| Cocoamidopropylbetain | --- | --- | 0,1 |
| Aroma | 0,5 | 0,5 | 0,5 |
| Ethanol | --- | 10 | 20 |
| Maleic anhydride, methyl vinyl ether copolymer | 0,1 | 0,25 | 0,5 |
| Wasser | ad 100 | ad 100 | ad 100 |

## Patentansprüche

1. Mund- und Zahnpflege- und -reinigungsmittel, enthaltend - bezogen auf sein Gewicht -
a) mindestens einen Extrakt aus *Quassia Amara,*
b) 500 bis 1600 ppm Fluorid.

2. Mund- und Zahnpflege- und -reinigungsmittel nach Anspruch 1, **dadurch gekennzeichnet, dass** es - bezogen auf sein Gewicht - 0,001 - 5 Gew.-%, vorzugsweise 0,001 - 4 Gew.-%, besonders bevorzugt 0,002 - 2 Gew.-%, außerordentlich bevorzugt 0,003 - 1 Gew.-% und insbesondere 0,005 bis 0,5 Gew.-% Extrakt(e) aus *Quassia Amara* enthält.

3. Mund- und Zahnpflege- und -reinigungsmittel nach einem der Ansprüche 1 oder 2, **dadurch gekennzeichnet, dass** es 1225 bis 1575 ppm, vorzugsweise 1250 bis 1550 ppm, weiter bevorzugt 1275 bis 1525 ppm, noch weiter bevorzugt 1300 bis 1500 ppm, noch weiter bevorzugt 1325 bis 1475 ppm und insbesondere 1350 bis 1450 ppm Fluorid enthält.

4. Mund und Zahnpflege- und -reinigungsmittel nach einem der Ansprüche 1 bis 3, **dadurch gekennzeichnet, dass** es - bezogen auf sein Gewicht - 0,27 bis 0,35 Gew.-%, vorzugsweise 0,28 bis 0,34 Gew.-%, weiter bevorzugt 0,29 bis 0,33 Gew.-% und insbesondere 0,30 bis 0,32 Gew.-% Natriumfluorid, enthält.

5. Mund und Zahnpflege- und -reinigungsmittel nach einem der Ansprüche 1 bis 4, **dadurch gekennzeichnet, dass** es 0,1 bis 5 Gew.-%, vorzugsweise 0,2 bis 4,0 Gew.-%, besonders bevorzugt 0,5 bis 3,5 Gew.-%, weiter bevorzugt 0,75 bis 3,0 Gew.-% und insbesondere 1 bis 2,5 Gew.-% nichtionische Tenside enthält.

6. Mund und Zahnpflege- und -reinigungsmittel nach einem der Ansprüche 1 bis 5, **dadurch gekennzeichnet, dass** es 0 bis 5 Gew.-%, vorzugsweise 0,1 bis 4,5 Gew.-%, besonders bevorzugt 0,5 bis 4 Gew.-%, weiter bevorzugt 0,75 bis 3,5 Gew.-% und insbesondere 1 bis 3 Gew.-% anionische Tenside enthält.

7. Verfahren zum Reinigen von Zähnen, **dadurch gekennzeichnet, dass** ein Mittel nach einem der Ansprüche 1 bis 6 auf den Bürstenkopf einer Zahnbürste aufgetragen und mit der Zahnbürste die Zähne geputzt werden.

8. Verfahren zur Zahnreinigung, **gekennzeichnet durch** die Schritte
a) Bereitstellung einer Zahnbürste, deren Bürstenkopf elektrisch in Bewegung versetzt werden kann;
b) Applikation von 0,5 bis 5 g eines Mittels nach einem der Ansprüche 1 bis 6 auf den Bürstenkopf,
c) 30- bis 300-sekündiges Zähneputzen mit dem Mittel nach einem der Ansprüche 1 bis 7 unter Einsatz des elektrisch in Bewegung versetzten Bürstenkopfes.

9. Nicht-therapeutische, kosmetische Verwendung von Extrakt aus *Quassia Amara*
- zur Steigerung der Collagensynthese in Gingiva- Fibroblasten und/oder
- zur Steigerung der ATP-Synthese von Gingiva-Keratinozyten und/oder
- zur Vitalisierung und Kräftigung des Zahnfleischs und der Mundschleimhaut und/oder
- zur Verbesserung der Zahnfleischregeneration und/oder
- zur Verjüngung des Zahnfleisches und/oder
- zum Schutz vor Zahnfleischentzündungen und/oder
- zur Glättung der Zahnschmelzoberflächen und/oder
- zur Reparatur mikroskopisch feiner Fissuren im Zahnschmelz und/oder
- zur Senkung der Zahnempfindlichkeit und/oder
- zur Senkung der Zahnempfindlichkeit gegen süße Speisen und Getränke und/oder
- zur Senkung der Zahnempfindlichkeit gegen saure Speisen und Getränke und/oder
- zur Senkung der Zahnempfindlichkeit gegen heiße Speisen und Getränke und/oder
- zur Senkung der Zahnempfindlichkeit gegen kalte Speisen und Getränke und/oder
- zur Senkung der Zahnempfindlichkeit gegen taktile Reize.

10. Nicht-therapeutische, kosmetische Verwendung eines Mund und Zahnpflege- und
- reinigungsmittels nach einem der Ansprüche 1 bis 6
- zur Steigerung der Collagensynthese in Gingiva- Fibroblasten und/oder
- zur Steigerung der ATP-Synthese von Gingiva-Keratinozyten und/oder
- zur Vitalisierung und Kräftigung des Zahnfleischs und der Mundschleimhaut und/oder
- zur Verbesserung der Zahnfleischregeneration und/oder
- zur Verjüngung des Zahnfleisches und/oder
- zum Schutz vor Zahnfleischentzündungen und/oder
- zur Glättung der Zahnschmelzoberflächen und/oder
- zur Reparatur mikroskopisch feiner Fissuren im Zahnschmelz und/oder
- zur Senkung der Zahnempfindlichkeit und/oder
- zur Senkung der Zahnempfindlichkeit gegen süße Speisen und Getränke und/oder
- zur Senkung der Zahnempfindlichkeit gegen saure Speisen und Getränke und/oder
- zur Senkung der Zahnempfindlichkeit gegen heiße Speisen und Getränke und/oder
- zur Senkung der Zahnempfindlichkeit gegen kalte Speisen und Getränke und/oder
- zur Senkung der Zahnempfindlichkeit gegen taktile Reize.
